Europäisches Patentamt

European Patent Office

Office européen des brevets

(19)

(11) **EP 1 218 535 B1**

(12) **EUROPÄISCHE PATENTSCHRIFT**

(45) Veröffentlichungstag und Bekanntmachung des Hinweises auf die Patenterteilung:
**30.11.2005 Patentblatt 2005/48**

(21) Anmeldenummer: **00956466.7**

(22) Anmeldetag: **19.08.2000**

(51) Int Cl.[7]: **C12Q 1/527**

(86) Internationale Anmeldenummer:
**PCT/EP2000/008102**

(87) Internationale Veröffentlichungsnummer:
**WO 2001/020023 (22.03.2001 Gazette 2001/12)**

(54) **VERFAHREN ZUM NACHWEIS DER LÖSLICHEN GUANYLATZYKLASE**

METHOD FOR THE DETECTION OF SOLUBLE GUANYLATE CYCLASE

PROCEDE POUR LA MISE EN EVIDENCE DE LA GUANYLATE-CYCLASE SOLUBLE

(84) Benannte Vertragsstaaten:
**AT BE CH CY DE DK ES FI FR GB GR IE IT LI LU MC NL PT SE**

(30) Priorität: **15.09.1999 DE 19944226**

(43) Veröffentlichungstag der Anmeldung:
**03.07.2002 Patentblatt 2002/27**

(73) Patentinhaber: **Sanofi-Aventis Deutschland GmbH**
**65929 Frankfurt am Main (DE)**

(72) Erfinder:
- **SCHINDLER, Ursula**
  **65812 Bad Soden (DE)**
- **STROBEL, Hartmut**
  **65835 Liederbach (DE)**
- **SCHINDLER, Peter**
  **65812 Bad Soden (DE)**
- **MÜLSCH, Alexander**
  **69115 Heidelberg (DE)**

(56) Entgegenhaltungen:
**WO-A-00/02851        DE-A- 19 837 015**
**SU-A- 876 715        US-A- 5 618 665**

- **MAKINO RYU ET AL: "EPR characterization of axial bond in metal center of native and cobalt-substituted guanylate cyclase." JOURNAL OF BIOLOGICAL CHEMISTRY, Bd. 274, Nr. 12, 19. März 1999 (1999-03-19), Seiten 7714-7723, XP002156928 ISSN: 0021-9258**

- **IGNARRO L J ET AL: "SELECTIVE ALTERATIONS IN RESPONSIVENESS OF GUANYLATE CYCLASE EC-4.6.1.2 TO ACTIVATION BY NITROSO COMPOUNDS DURING ENZYME PURIFICATION" BIOCHIMICA ET BIOPHYSICA ACTA, Bd. 673, Nr. 4, 1981, Seiten 394-407, XP000980629 ISSN: 0006-3002**
- **OLESEN SOREN-PETER ET AL: "Characterization of NS 2028 as a specific inhibitor of soluble guanylyl cyclase." BRITISH JOURNAL OF PHARMACOLOGY, Bd. 123, Nr. 2, Januar 1998 (1998-01), Seiten 299-309, XP000980198 ISSN: 0007-1188 in der Anmeldung erwähnt**
- **SCHRAMMEL ASTRID ET AL: "Characterization of 1H-(1,2,4)Oxadiazolo(4,3-a)quinoxalin-1-on e as a heme-site inhibitor of nitric oxide-sensitive guanylyl cyclase." MOLECULAR PHARMACOLOGY, Bd. 50, Nr. 1, 1996, Seiten 1-5, XP000980673 ISSN: 0026-895X in der Anmeldung erwähnt**
- **GUPTE SACHIN A ET AL: "NADPH and heme redox modulate pulmonary artery relaxation and guanylate cyclase activation by NO." AMERICAN JOURNAL OF PHYSIOLOGY, Bd. 277, Nr. 6 part 1, Dezember 1999 (1999-12), Seiten L1124-L1132, XP002157463 ISSN: 0002-9513**

**Beschreibung**

Die Erfindung betrifft ein Verfahren zum Nachweis von löslicher Guanylatzyklase.

Lösliche Guanylatzyklasen sind heterodimere Proteine. Sie bestehen aus je einer $\alpha$- und einer $\beta$-Untereinheit und enthalten Häm als prosthetische Gruppe. Die Bindung des Signalmoleküls NO an das Häm aktiviert das Enzym. Das durch enzymatische Aktivität der löslichen Guanylatzyklase gebildete cGMP ist unter anderem beteiligt an der Aktivierung cGMP abhängiger Proteinkinasen, an der Regulierung von Phosphodiesterasen oder von Ionenkanälen. Für die Untereinheiten wurden vier Isoformen beschrieben. Diese unterscheiden sich hinsichtlich ihrer Sequenz sowie der gewebespezifischen und entwicklungsspezifischen Expression. Die Subtypen $\alpha_1$ und $\beta_1$ findet man vorwiegend in Lunge, Niere und Gehirn. Die $\beta_2$-Kette wird überwiegend in Leber und Niere und $\alpha_2$ in der Plazenta exprimiert. Eine Dimerisierung der Untereinheiten ist Voraussetzung für eine katalytisch aktive lösliche Guanylatzyklase. Bekannt sind die Heterodimere $\alpha_1/\beta_1$, $\alpha_2/\beta_1$ und $\alpha_1/\beta_2$. Bei allen Untereinheiten ist eine starke Homologie im Bereich der katalytischen Domäne zu beobachten.

Die lösliche Guanylatzyklase (sGC) enthält ein Häm pro Heterodimer. Die Bindung des Häms erfolgt über das His-105 der $\beta_1$-Kette. Mutanten, die im N-Terminus der $\beta_1$ Untereinheit kein His-105 mehr enthalten, sind nicht mehr durch NO stimulierbar. Das Häm der löslichen Guanylatzyklase besteht aus einem organischen Molekülteil und einem Eisenatom. Der organische Teil, das Protoporphyrin IX, enthält vier Pyrrolringe, die durch Methinbrücken zu einem Tetrapyrrolsystem verknüpft sind. Das Eisenatom im Häm ist an vier Stickstoffatome im Zentrum des Protoporphyrinringes gebunden. Es kann darüber hinaus zwei weitere Bindungen eingehen. Das Eisen des Häms kann in der Oxidationsstufe +2 (Ferroform) oder +3 (Ferriform; oxidierte Form) vorliegen. Die Oxidationsstufe des Eisens im Häm hat entscheidenden Einfluß auf die enzymatische Funktion der löslichen Guanylatzyklase. Das Enzym mit dem Hämeisen in dreiwertiger Form zeigt wie eine lösliche Guanylatzyklase ohne Hämgruppe nur basale enzymatische Aktivität und ist nicht durch NO stimulierbar. Die Herstellung einer hämfreien löslichen Guanylatzyklase wird in Eur. J. Biochem. 240, 380-386 (1996) beschrieben.

Lösliche Guanylat-Cyclase (sGC) katalysiert die Umwandlung von GTP in zyklisches Guanosinmonophosphat (cGMP) und Pyrophosphat. cGMP funktioniert als intrazellulärer Botenstoff (second messenger). Second messenger entstehen im Zellinnern in kaskadenartigen Reaktionen. Ihr Spiegel wird von extrazellulären Signalen (z.B. Hormone, Neurotransmitter, Wachstumsfaktoren, Geruchsstoffe, Peptide, Licht) kontrolliert. Die Bildung von Second messengern dient der Signalverstärkung. Der Second messenger übermittelt in der Zelle Signale an bestimmte, vom Zelltyp abhängige Zielproteine (Kinasen, Phosphatasen, Ionenkanäle und andere). Die Modulation der löslichen Guanylatzyklase führt deshalb über die Beeinflussung der cGMP-Spiegel und damit gesteuerter Zielproteine zu einer Reihe von pharmakologischen Effekten. Beispiele für solcherart beeinflußte Mechanismen sind die Entspannung der glatten Muskulatur (z.B. in den Wänden der Blutgefäße), die Inhibierung der Thrombozytenaktivierung, die Hemmung der Vermehrung von glatten Muskelzellen oder die Adhäsion von Leukozyten.

Lösliche Guanylatzykase ist in Organen wie beispielsweise Herz, Lunge, Leber, Niere, Gehirn bei allen Säugetieren einschließlich des Menschen nachweisbar. In pathologischen oder für Krankheitsgeschehen relevanten Vorgängen kann die Oxidationsstufe des Hämeisens der löslichen Guanylatzyklase eine wesentliche Rolle spielen. Ein höherer Anteil von löslicher Guanylatzyklase mit oxidiertem Hämeisen hätte eine geringere Aktivierbarkeit der löslichen Guanylatzyklase durch endogenes NO zur Folge. Dies könnte unter anderem über Anstieg des Blutdrucks, Aktivierung von Plättchen, vermehrte Proliferation von Zellen oder verstärkte Adhäsion von Zellen zu dauerhaftem Bluthochdruck, stabiler oder instabiler Angina pectoris, Thrombosen, Myokardinfarkt, Schlaganfällen, Lungenödemen, erektiler Dysfunktion, unkontrolliertem Gewebewachstum mit Tumorbildung, Diabetes, Nierenfunktionsstörungen, Leberfunktionsstörungen oder vaskulärer Dysfunktion führen.

Die Endothelzellen von Gefäßwänden sezernieren NO als parakrines Hormon unter anderem zur Aktivierung der löslichen Guanylatzyklase. Zur pharmakologischen Stimulierung der löslichen Guanylatzyklase werden häufig Verbindungen verwendet, die über eine intermediäre NO-Freisetzung als NO Donoren wirken. Beispiele dafür sind die organischen Nitrate. Darüberhinaus sind verschiedene nicht über eine NO-Freisetzung wirkende Verbindungen beschrieben, welche die Aktivität der löslichen Guanylatzyklase modifizieren.

Die Aktivierung der löslichen Guanylatzyklase durch NO-Donoren bzw. freies NO erfolgt ausschließlich im reduzierten d. h. ($Fe^{2+}$)-haltigen Zustand des Hämeisens. Dies geht aus Versuchen hervor, die von A. Schrammel et al. in Mol. Pharmacol. 50, 1 (1996) mit $1$H-[1,2,4] oxadiazolo [4,3-a]quinoxalin-1-one (=ODQ) durchgeführt wurde. ODQ ist ein spezifischer und hochwirksamer Hemmstoff der löslichen Guanylatzyklase. ODQ interagiert mit der prosthetische Hämgruppe. Der Stoff bewirkt in vitro eine irreversible Oxidation des Hämeisens der löslichen Guanylatzyklase. Die Behandlung der löslichen Guanylatzyklase mit ODQ hat zur Folge, daß die stimulierende Wirkung von NO auf das Enzym verloren geht. Die Oxidation des Hämeisens der löslichen Guanylatzyklase kann auch mit Oxadiazolo(3,4-d) benz(b)(1,4)oxazin-1-one (Olesen et al., British Journal of Pharmacology 123, 299 - 309 (1998)) oder Kaliumferricyanid (Koesling et al., in "Reviews of Physiology Biochemistry and Pharmacology" S. 41 - 65, Springer Verlag 1999) bewirkt werden. Es gibt auch Aktivatoren der löslichen Guanylatzyklase, die nicht über eine NO-Freisetzung wirken. Solche wurden beispielsweise von Vesely et al., in Eur. J. Clin. Invest. 15, 258 (1985) beschrieben. Eine Stimulation hämfreier löslicher Guanylatzyklase durch Protoporphyrin IX ist durch Ignarro et al. in Adv. Pharmacol. 26, 35 (1994) nachgewiesen. Die Wirkung von Protoporphyrin IX ist durch ODQ nicht hemmbar, sondern wird durch die Verbindung verstärkt (Koesling und Friebe in Physiol. Biochem. Pharmacol.135, 41 (1999)). Die bisher bekannten Aktivatoren der löslichen Guanylatzyklase stimulieren die enzymatische Aktivität dieser nur, wenn das Hämeisen im reduzierten d.h. $Fe^{2+}$-haltigen Zustand vorliegt.

Mit den schwefelsubstituierten Sulfonylamino-carbonsäure-N-arylamiden wurde kürzlich eine weitere chemische Verbindungsklasse beschrieben (WO 00/02851), deren Vertreter lösliche Guanylatzyklase aktivieren können.

Der Nachweis der Aktivität der löslichen Guanylatzyklase erfolgt bisher beispielsweise mittels enzymatischer Methoden zum Nachweis von cGMP (z.B. in US 5 618 665 A) sowie cAMP oder durch photometrische Verfahren zum Nachweis der Hämgruppe.

Zur Feststellung der Abhängigkeit eines krankheitsmäßig veränderten Zustands vom Zustand der löslichen Guanylatzyklase, genügt es nicht, das Protein mittels immunologischer Nachweisverfahren oder der Verwendung von Markierungstechniken lediglich nachzuweisen. Es ist ebenso wichtig, den Redoxzustand des komplexierten Eisens der Hämgruppe zu kennen. Eine Aussage über die Funktionalität des Häms der löslichen Guanylatzyklase ist bisher beispielsweise über Bestimmung des Redoxzustands des komplexierten Hämeisens mittels ESR-Messungen oder photometrisch feststellbar. Diese Bestimmungen haben den Nachteil, daß sie von der Verfügbarkeit einer teilweise komplexen technischen Apparatur abhängen. Die Methoden eignen sich darüberhinaus nur eingeschränkt für spezifische Messungen, weil sie durch Verunreinigungen durch andere hämhaltige Proteine leicht zu stören sind.

Eine Aufgabe der vorliegenden Erfindung bestand deshalb darin, ein vereinfachtes und spezifisches Verfahren zum Nachweis der löslichen Guanylatzyklase bereitzustellen.

Die vorliegende Erfindung betrifft einen luminometrischen Test zum Nachweis einer löslichen Guanylatzyklase umfassend die Verfahrensschritte

a] Bereitstellung einer löslichen Guanylatzyklase und GTP als Substrat und Umsetzung des GTP zu cGMP und Pyroposphat;

b] Bereitstellung einer Nicotinamid-Mononukleotid-Adenyltransferase und Nicotinamiddinukleotid ($NAD^+$) und Umsetzung mit dem Pyrophosphat aus a] zu ATP;

c] Bereitstellung von Luziferase und Luziferin und luminometrische Bestimmung des in b] gebildeten ATP durch Umsetzung mit Luziferin und Luziferase.

Die lösliche Guanylatzyklase kann aus einer Mischung aus Molekülen der löslichen Guanylatzyklase bestehen, wobei nicht alle lösliche Guanylatzyklasemoleküle mit einem Hämeisen in der dreiwertigen Oxidationsstufe vorliegen. Insbesondere kann sich ein Teil der Hämgruppen der löslichen Guanylatzyklasemoleküle in der dreiwertigen Oxidationsstufe und ein anderer Teil in der zweiwertigen Oxidationsstufe befinden, so daß sich ein Gemisch mit unterschiedlichem Anteil der Oxidationszustände des Hämeisens ergibt. Das Verfahren zum Nachweis einer löslichen Guanylatzyklase, deren Hämeisen in dreiwertiger Form vorliegt, kann beispielsweise im Vergleich zu Referenzwerten durchgeführt werden. Die Referenzwerte können unter anderem beispielsweise aus Versuchen gewonnen werden, in denen die Abhängigkeit der Aktivität der löslichen Guanylatzyklase von bekannten Anteilen an löslicher Guanylatzyklase mit dreiwertigem Hämeisen ermittelt wurde. Ein Maß für den jeweiligen Anteil von löslicher Guanylatzyklase mit dreiwertigem Hämeisen kann durch die Vorbehandlung der löslichen Guanylatzyklase mit ODQ und anschließender Bestimmung der Aktivität dieser löslichen Guanylatzyklase gewonnen werden. Die ermittelten Referenzwerte können dann als Eichkurven für quantitative Bestimmungen der löslichen Guanylatzyklase mit Hämeisen in der dreiwertigen Oxidationsstufe verwendet werden.

Die lösliche Guanylatzyklase kann in unterschiedlichen Zustandsformen, beispielsweise als unterschiedliche heterodimere lösliche Guanylatzyklase, verwendet werden. Grundsätzlich kann eine lösliche Guanylatzyklase jeder Spezies, insbesondere jeder Säugetierspezies eingesetzt werden. Vorzugsweise wird lösliche Guanylatzyklase aus Rindern verwendet, die bevorzugt aus dem Lungengewebe isoliert wird. Besonders bevorzugt wird humane lösliche Guanylatzyklase verwendet. Die bereitgestellte lösliche Guanylatzyklase kann bevorzugt aus je einer $\alpha$-Untereinheit, beispielsweise der $\alpha_1$- oder $\alpha_2$-Untereinheit und einer $\beta$-Untereinheit beispielsweise der $\beta_1$- oder $\beta_2$-Untereinheit zusammengesetzt sein. Für das Verfahren der vorliegenden Erfindung können vorzugsweise Untereinheiten der löslichen Guanylatzyklase verwendet werden. Entsprechende Sequenzinformationen sind in Giuili et al. (FEBS Letters 304, 83 - 88 (1992)) für die zwei Untereinheiten der löslichen Guanylatzyklase ursprünglich isoliert aus menschlichem Gehirn oder in Nakane et al. (Journal of Biol. Chem. 265, 16841 -16845 (1990)) für cDNAs der löslichen Guanylatzyklase aus Lungengewebe von Ratten oder in Yuen et al. (Biochemistry 29, 10872 - 10878 (1990)) für eine lösliche Guanylatzyklase aus Nieren von Ratten angegeben.

Die Bereitstellung einer löslichen Guanylatzyklase kann durch Isolierung mittels biochemischer Methoden aus einem biologischen Material erfolgen. Insbesondere in solchen Fällen liegt häufig eine Mischung von löslichen Guanylatzyklasemolekülen mit unterschiedlichen Oxidationszuständen des Hämeisens vor. Biochemische Methoden können unter anderem sein: Aufschlußmethoden für das biologische Material, Zentrifugation, chromatographische Trennungen, Gelelektrophoresen, isoelektrische Fokussierung, immunologische Methoden. Biologisches Material kann eukaryotische Zellen oder prokaryotische Zellen, Aufschlüsse der Zellen oder Zubereitungen aus Aufschlüßen der Zellen, ganzen Zellen oder Teile oder Fraktionen der Zellen enthalten. Zu den eukaryotischen Zellen zählen unter anderem beispielsweise Zellen aus Geweben oder Organen wie Herz, Blutgefäße, Lunge, Blut, Gehirn, Leber, Niere, Fettgewebe, Muskel von Wirbeltieren einschließlich des Menschen, Gewebe- oder Blutproben aber auch Zellen aus humanen oder tierischen Zellkulturen sowie Insektenzellkulturen. Als spezielle Beispiele seien Thrombozyten genannt, die aus menschlichen oder tierischen Blutproben gewonnen werden können, sowie glatte Muskelzellen, die man beispielsweise aus Blutgefäßen tierischen oder humanen Ursprungs isolieren kann. Weitere Beispiele sind Biopsiematerial, übriggebliebene Organe und Gewebe und Teile von diesen aus Transplantationen, Nabelschnüre oder Plazenten. Prokaryotische Zellen können beispielsweise Bakterien unter anderem Escherichia coli, Salmonella typhimurium, Bacillus subtilis, ebenso Pilze wie Saccharomyces cerevisiae, Saccharomyces pombe oder Pichia pastoris bedeuten. In einer besonderen Ausführungsform der Erfindung kann die lösliche Guanylatzyklase rekombinant hergestellt werden z.B. durch Expression in eukaryotischen oder prokaryotischen Zellen, wobei die lösliche Guanylatzyklase mit oder ohne prosthetische Hämgruppe mittels Expressionsvektoren innerhalb der Zellen angereichert oder ins Medium ausgeschieden wird. In einer speziellen Ausführungsform wird die lösliche Guanylatzyklase nach einem Verfahren wie in Humbert P. et al., Methods of Enzymology 195, 384-391 (1991) beschrieben isoliert.

In einer Ausführungsform wird eine lösliche Guanylatzyklase zur Verfügung gestellt, die mit einem Oxidationsmittel behandelt wurde. In einer bevorzugten Ausführungsform wird eine lösliche Guanylatzyklase zur Verfügung gestellt, die mit ODQ behandelt wurde. Die Konzentration des ODQ beträgt z.B. 0,001 mM bis 0,5 mM, vorzugsweise 0,005 mM bis 0,1 mM und besonders bevorzugt 0,01 mM. Es kann auch bevorzugt eine lösliche Guanylatzyklase zur Verfügung gestellt werden, die mit Oxadiazolo(3,4-d)benz(b)(1,4)oxazin-1-one oder einem anderen Derivat von ODQ behandelt wurde. In einer weiteren bevorzugten Ausführungsform wird eine lösliche Guanylatzyklase zur Verfügung gestellt, die mit Kaliumferricyanid behandelt wurde. Die Behandlung der löslichen Guanylatzyklase mit Oxidationsmitteln und/oder ODQ und/oder anderen Verbindungen kann eine Oxidation des Hämeisens, also eine Überführung des Eisens aus der zweiwertigen Oxidationsstufe ($Fe^{2+}$) in die dreiwertige Oxidationsstufe ($Fe^{3+}$) in Abhängigkeit von den gewählten Bedingungen entweder nur bei einem Teil oder in besonderen Ausführungsformen bei allen behandelten löslichen Guanylatzyklasemolekülen bewirken. Generell kann zur Durchführung des Verfahrens der vorliegenden Erfindung eine Form der löslichen Guanylatzyklase verwendet werden, bei der entweder nur bei einem Teil der löslichen Guanylatzyklasemoleküle oder in einer bevorzugten Ausführungsform bei allen löslichen Guanylatzyklasemolekülen das Hämeisen in der dreiwertigen Oxidationsstufe vorliegt.

Die Bereitstellung der löslichen Guanylatzyklase umfaßt die Zubereitung geeigneter Formen für die Inkubation mit bereitgestellten chemischen Verbindungen, wobei Inkubation bedeutet, daß die lösliche Guanylatzyklase und die chemische Verbindung miteinander in Kontakt gebracht werden. Das Protein kann beispielsweise in wäßrigen Lösungsmitteln ergänzt durch Puffer, Ionen oder auch Hilfsreagenzien suspendiert oder gelöst werden. Es kann beispielsweise auch auf Trägermaterial aufgebracht werden und in dieser Form fixiert suspendiert in Lösungsmitteln verwendet werden.

Als chemische Verbindungen zur Stimulierung der Aktivität der löslichen Guanylatzyklase mit dreiwertigem Hämeisen können einzelne chemische Verbindungen oder auch Kombinationen von chemischen Verbindungen bereitgestellt werden. Die chemischen Verbindungen können z.B. synthetisiert oder als Naturstoff gewonnen sein. Es können grundsätzlich alle chemischen Verbindungen zur Bereitstellung verwendet werden, die die Aktivität einer löslichen Guanylatzyklase stimulieren, wenn das Eisen des Häms dieser löslichen Guanylatzyklase in der dreiwertigen Oxidationsstufe vorliegt.

Zur Ausführung des Verfahrens zum Nachweis der löslichen Guanylatzyklase mit einem Hämeisen in der dreiwertigen Oxidationsstufe kann beispielsweise die chemische Verbindung 2-(4-Chlor-phenylsulfonylamino)-4,5-dimethoxy-N-(4-(thiomorpholin-4-sulfonyl)-phenyl)-benzamid verwendet werden.

Zur Durchführung des Verfahrens zum Nachweis einer löslichen Guanylatzyklase mit einem dreiwertigen Hämeisen können chemische Verbindungen verwendet werden, die ausschließlich die lösliche Guanylatzyklase mit dreiwertigem Hämeisen stimulieren. Ebenso können Verbindungen verwendet werden, die neben der Aktivierung der löslichen Guanylatzyklase mit einem dreiwertigen Hämeisen noch andere Wirkungen auf die lösliche Guanylatzyklase zeigen. Beispielsweise können chemische Verbindungen verwendet werden , die neben der Aktivierung der löslichen Guanylatzyklase mit einem dreiwertigen Hämeisen auch eine Aktivierung der basalen Aktivität der löslichen Guanylatzyklase oder eine Aktivierung der löslichen Guanylatzyklase mit einem zweiwertigem Hämeisen bewirken. Ein Verfahren zum Nachweis der löslichen Guanylatzyklase mittels chemischer Verbindungen, die ausschließlich die Enzymform mit dreiwertigem Hämeisen oder beispielsweise auch Enzymformen mit zweiwertigem Hämeisen aktivieren, erfolgt unter Zuhilfenahme von Referenzwerten, Eichkurven und/oder im Vergleich mit Substanzen, die ausschließlich eine lösliche Guanylatzyklase mit zweiwertigem Hämeisen (z.B. NO-Donoren) aktivieren können. Zur Erstellung einer Eichkurve kann die lösliche Guanylatzyklase mit unterschiedlichen Konzentrationen von z.B. ODQ inkubiert werden. In Abhängigkeit von der jeweils eingesetzten ODQ-Konzentration stellt sich ein bestimmtes Verhältnis von löslicher Guanylatzyklase mit dreiwertigem zu löslicher Guanylatzyklase mit zweiwertigem Hämeisen ein. Die verwendete ODQ Konzentration kann direkt als Maß für den Anteil von Enzym mit dreiwertigem Hämeisen verwendet werden. Der absolute Anteil an löslicher Guanylatzyklase mit dreiwertigem Hämeisen läßt sich zu Eichzwecken durch andere Meßverfahren beispielsweise durch ESR-Messungen überprüfen. Die mit ODQ unterschiedlich vorbehandelten löslichen Guanylatzyklasen werden durch die chemische Verbindungen in einem Inkubationsschritt aktiviert. Dabei ergeben Verbindungen, welche ausschließlich eine lösliche Guanylatzyklase mit dreiwertigem Hämeisen aktivieren, einen Wert, der direkt proportional zur Menge an löslicher Guanylatzyklase mit dem dreiwertigen Hämeisen ist. Bei der Verwendung von chemischen Verbindungen, die neben der löslichen Guanylatzyklase mit dreiwertigem Hämeisen auch noch die Form mit zweiwertigem Hämeisen aktivieren, wird eine zusätzliche Eichkurve mit einer Substanz benötigt, die eine lösliche Guanylatzyklase ausschließlich in der zweiwertigen Form aktiviert. Der Anteil an löslicher Guanylatzyklase mit dreiwertigem Hämeisen ergibt sich dann durch Vergleich der Aktivitäten, die nach unterschiedlicher Stimulierung einer mit ODQ vorbehandelten löslichen Guanylatzyklase erhalten wird. Dabei wird die lösliche Guanylatzyklase einmal mit einer chemischen Verbindung stimuliert, die ausschließlich lösliche Guanylatzyklase mit zweiwertigem Hämeisen aktiviert, und zum anderen wird mit einer chemischen Verbindung stimuliert, die lösliche Guanylatzyklase mit dreiwertigem sowie zweiwertigem Hämeisen aktiviert. Die Bestimmung des Anteils an löslicher Guanylatzyklase mit dreiwertigem Hämeisen in einer zu untersuchenden biologischen Probe ergibt sich durch Vergleich der Aktivität der löslichen Guanylatzyklase aus dieser Probe nach Inkubation mit einer chemischen Verbindung, welche die lösliche Guanylatzyklase wie oben beschrieben aktivieren kann, mit den Werten der Eichkurve.

Die Bereitstellung einer chemischen Verbindung kann auch die Lösung dieser Verbindung, in geeigneten Lösungsmitteln wie beispielsweise Dimethylsulfoxid (DMSO) oder Wasser sowie die Zubereitung einer Formulierung mit Hilfsreagenzien umfassen.

Die Inkubation kann unterschiedlich lange Zeit in Anspruch nehmen und bei verschiedenen Temperaturen vorgenommen werden. Zeitbedarf und Temperatureinstellung hängen von den im jeweiligen Fall gewählten Ausführungsformen sowohl hinsichtlich der Auswahl der bereitgestellten löslichen Guanylatzyklase, der bereitgestellten chemischen Verbindung und/oder auch anderer Bedingungen, wie der Zubereitungsform, der Konzentration der eingesetzten löslichen Guanylatzyklase und/oder der eingesetzten chemischen Verbindung sowie anderer Zusatzstoffe ab. Der Zeitraum für die Inkubation kann beispielsweise zwischen 1 Minute und 120 Minuten betragen, vorzugsweise beträgt der Zeitraum für die Inkubation zwischen 1 Minute und 60 Minuten und besonders bevorzugt wird ein Zeitraum von 30 Minuten gewählt. Die Temperatur für die Inkubation kann zwischen 5°C und 45°C liegen, bevorzugt liegt die Temperatur zwischen 20°C und 42°C und in der besonders bevorzugten Ausführungsform bei 37°C.

Der Test umfaßt mehrere Verfahrensschritte. Die Verfahrensschritte können nacheinander oder gleichzeitig durchgeführt werden. Der luminometrische Test besteht aus den folgenden Schritten: a) der Bereitstellung einer löslichen Guanylatzyklase und GTP als Substrat, b) der Umsetzung des GTP mittels der bereitgestellten löslichen Guanylatzyklase wodurch cGMP und Pyrophosphat gebildet werden, c) der Bereitstellung einer Nicotinamid-Mononukleotid-Adenyltransferase und Nicotinamiddinukleotid ($NAD^+$), d) der Umsetzung des in b) gebildeten Pyrophosphats mit der Nicotinamid-Mononukleotid-Adenyltransferase in Anwesenheit von Nicotinamiddinukleotid ($NAD^+$), wobei ATP gebildet wird, e) der Bereitstellung einer Luziferase und deren Substrat Luziferin und f) der luminometrischen Bestimmung des gebildeten ATPs durch Umsetzung mit Luziferin unter Katalyse von Luziferase. Die Menge an gebildetem ATP ist proportional zur Konzentration an löslicher Guanylatzyklase. Das Verfahren wird gestartet durch Zugabe von löslicher Guanylatzyklase in einen Reaktionsansatz, der neben anderen Substanzen GTP als Substrat enthält. Andere Substanzen können beispielsweise Puffer, Ionen oder Proteine sein. Das Verfahren zum Nachweis der löslichen Guanylatzyklase eignet sich in einer bevorzugten Ausführungsform zur Durchführung eines Screening von zu untersuchenden chemischen Verbindungen auf deren Eignung, die Aktivität der löslichen Guanylatzyklase zu stimulieren. Der Vorteil dieses luminometrischen Verfahrens gegenüber den bisher verwendeten Nachweisverfahren für die lösliche Guanylatzyklase (immunologische, enzymatische, photometrische Verfahren) besteht in der Möglichkeit, große Mengen an zu untersuchenden chemischen Verbindungen in wesentlich kürzeren Zeitabschnitten auf deren potentielle Fähigkeit zur Stimulierung der Aktivität einer löslichen Guanylatzyklase mit dreiwertigem Hämeisen screenen zu können. Zur Durchführung des Screenings wird die zu untersuchende chemische Verbindung vor dem Start des Verfahrens durch Zugabe der löslichen Guanylatzyklase in den Reaktionsansatz gegeben. Das Verfahren eignet sich in einer besonderen Ausführungsform des Screenings zur Anwendung mittels eines Laborroboters und in einer weiteren besonders bevorzugten Ausführungsform zur manuellen Durchführung. Die Bereitstellung der löslichen Guanylatzyklase kann wie in den vorhergehenden Abschnitten für die lösliche Guanylatzyklase bereits ausgeführt erfolgen. Ein besonderer Vorteil dieses luminometrischen Tests besteht darin, daß die Komponenten zur Durchführung des Verfahrens wie GTP, Nicotinamid-Mononukleotid-Adenyltransferase, $NAD^+$, Luziferin oder Luziferase in einem Reaktionsgefäß bereitgestellt werden können. Die Aktivität der löslichen Guanylatzyklase wird durch Bestimmung der Menge gebildetem an ATP ermittelt. Der Start der Reaktion erfolgt durch Zugabe von löslicher Guanylatzyklase. Dadurch wird es möglich, das Verfahren in einer Eintopfreaktion durchzuführen. Dies läßt sich auch auf ein Screening mit hohem Durchsatz an zu untersuchenden chemischen Substanzen (High Throughput Screening; HTS) anwenden und reproduzierbar durchführen. Dieser luminometrische Test ist insbesondere zum Nachweis geringer Mengen von Pyrophosphat sehr geeignet. Das Verfahren kann in einer bevorzugten Ausführungsform zur Bestimmung der Aktivität einer löslichen Guanylatzyklase verwendet werden. Der luminometrische Test eignet sich auch zur Durchführung eines wie in den vorhergehenden Abschnitten beschriebenen Verfahrens zum Nachweis der löslichen Guanylatzyklase mit dreiwertigem Hämeisen, wobei das Verfahren die folgenden Verfahrensschritte enthält:

a) Bereitstellung einer löslichen Guanylatzyklase;
b) Bereitstellung mindestens einer chemischen Verbindung, welche die Aktivität einer löslichen Guanylatzyklase stimuliert, wenn das Eisen des Häms dieser löslichen Guanylatzyklase in der dreiwertigen Oxidationsstufe vorliegt;
c) Inkubation einer löslichen Guanylatzyklase, bereitgestellt gemäß a), mit einer chemischen Verbindung, bereitgestellt gemäß b);
d) Bestimmung der Aktivität der löslichen Guanylatzyklase nach Inkubation gemäß c).

Eine lösliche Guanylatzyklase ist durch die bisher offenbarten pharmazeutischen Wirkstoffe nicht mehr aktivierbar, wenn sich ihr Hämeisen im dreiwertigen Zustand befindet. Chemische Verbindungen, welche die lösliche Guanylatzyklase aktivieren können, wenn deren Hämeisen dreiwertig oxidiert vorliegt, sind bisher nicht bekannt. Solche chemischen Verbindungen könnten als pharmazeutische Wirkstoffe in Arzneimittel zum Stimulieren der löslichen Guanylatzyklase verwendet werden, z.B. bei einem pathologischen Zustand hervorgerufen durch eine lösliche Guanylatzyklase mit einem dreiwertigen Hämeisen. Ebenso könnten solche Verbindungen in dem Verfahren zum Nachweis oxidierter Formen der löslichen Guanylatzyklase, wie erfindungmäßig beschrieben, verwendet werden. Eine andere Ausführungsform der Erfindung betrifft deshalb ein Verfahren zum Auffinden einer chemischen Verbindung, welche die Aktivität einer löslichen Guanylatzyklase stimuliert, wenn das Eisen des Häms der löslichen Guanylatzyklase in der dreiwertigen Oxidationsstufe vorliegt, wobei das Verfahren die folgenden Verfahrensschritte enthält:

a) Bereitstellung einer löslichen Guanylatzyklase, wobei das Eisen des Häms dieser löslichen Guanylatzyklase in der dreiwertigen Oxidationsstufe vorliegt;
b) Bereitstellung mindestens einer zu untersuchenden chemischen Verbindung;
c) Inkubation einer löslichen Guanylatzyklase gemäß a) mit mindestens einer zu untersuchenden chemischen Verbindung gemäß b) und
d) Bestimmung der Aktivität der löslichen Guanylatzyklase nach Inkubation gemäß c), gemäß dem luminometrischen Test.

Ein Verfahren zum Auffinden einer chemischen Verbindung wird auch als Screening bezeichnet. Verwendung und Bereitstellung der löslichen Guanylatzyklase für das Screeningverfahren erfolgt wie bereits beim Verfahren zum Nachweis einer löslichen Guanylatzyklase mit dreiwertigem Hämeisen ausgeführt.

Die Bereitstellung der löslichen Guanylatzyklase für das Screening kann die Zubereitung geeigneter Formen für die Inkubation mit einer zu untersuchenden chemischen Verbindung einschließen. Die lösliche Guanylatzyklase kann beispielsweise in wäßrigen Lösungsmitteln ergänzt durch Puffer, Ionen oder auch Hilfsreagenzien suspendiert oder gelöst werden. Sie kann beispielsweise auch auf Trägermaterial aufgebracht werden und in dieser Form fixiert suspendiert in Lösungsmitteln verwendet werden.

Bei der im Screening zu untersuchenden chemischen Verbindung kann es sich beispielsweise um eine chemisch synthetisierte Verbindung oder um einen Naturstoff handeln. Die zu untersuchende chemische Verbindung kann in unterschiedlichen Zustandsformen, beispielsweise als Einzelsubstanz in gereinigter Form, als Gemisch neben anderen Verbindungen, die nicht genau charakterisiert zu sein brauchen, als Gemisch verschiedener aktiver Verbindungen, als Bestandteil eines Verbindungsgemisches biologischen Ursprungs oder zusammen mit eukaryotischen und/oder prokaryotischen Organismen vorliegen. Das Verfahren zum Screening kann beispielsweise mittels eines Laborroboters oder unter Zuhilfenahme von Maschinen erfolgen. Zu untersuchende chemische Verbindungen, welche eine lösliche Guanylatzyklase mit einem dreiwertigen Hämeisen aktivieren, können als pharmazeutische Wirkstoffe verwendet werden zur Behandlung krankheitsmäßig veränderter Zustände wie beispielsweise Krebs, Angina pectoris, Diabetes, Myokardinfarkt, erektile Dysfunktion, Herzinsuffizienz, Bluthochdruck, Thrombosen, Anämie oder vaskuläre Dysfunktion. Die Erfindung eröffnet auch den Zugang zu einer chemischen Verbindung, die mittels dieses Screeningverfahrens als Aktivator einer löslichen Guanylatzyklase, deren Hämeisen in der dreiwertigen Oxidatiosstufe vorliegt, identifiziert wird. Die Erfindung eröffnet ganz allgemein den Zugang zu Verbindungen, welche die Aktivität einer löslichen Guanylatzyklase, deren Hämeisen in der dreiwertigen Oxidationsstufe vorliegt, aktivieren können und die Verwendung dieser Substanzen, vorzugsweise als pharmazeutische Wirkstoffe zur Behandlung oder Prävention pathologischer Zustände, die durch lösliche Guanylatzyklase mit Hämeisen in der dreiwertigen Oxidationsstufe verursacht oder verstärkt werden, eingesetzt werden können. Zu solchen pathologischen Zuständen können beispielsweise Krebs, Angina pectoris, Diabetes, Myokardinfarkt, erektile Dysfunktion, Herzinsuffizienz, Bluthochdruck, Thrombosen, vaskuläre Dysfunktion oder Anämie gehören.

Die Erfindung offenbart auch ein Diagnostikum zur Durchführung eines Verfahrens zur Bestimmung der Oxidationsstufe des Hämeisens einer löslichen Guanylatzyklase. Das Diagnostikum kann dazu mindestens eine oder mehrere der folgenden Komponenten enthalten: a) mindestens eine chemische Verbindung, welche die Aktivität der löslichen Guanylatzyklase stimuliert, wenn das Hämeisen dieser löslichen Guanylatzyklase in der dreiwertigen Oxidationsstufe vorliegt, sowie b) Lösungen und/oder Reagenzien zur Aktivitätsbestimmung einer löslichen Guanylatzyklase. Als chemische Verbindung in dem Diagnostikum kann beispielsweise 2-(4-Chlor-phenylsulfonylamino)-4,5-dimethoxy-N-(4-(thiomorpholin-4-sulfonyl)-phenyl)-benzamid verwendet werden. Die Bestimmung der Aktivität erfolgt durch den luminometrischen Test.

Das Diagnostikum kann beispielsweise auch lösliche Guanylatzyklase, eine chemische Verbindung, die eine lösliche Guanylatzyklase nur dann aktiviert, wenn das Hämeisen zweiwertig vorliegt, ein Oxidationsmittel und/oder ODQ enthalten. Diese Bestandteile können einzeln oder in Kombination zur Ermittlung eines Referenzwertes oder einer Eichkurve zur quantitativen oder qualitativen Bestimmung der löslichen Guanylatzyklase mit einem Hämeisen in der dreiwertigen Oxidationsstufe verwendet werden.

Das Diagnostikum kann beispielsweise zur Bestimmung des Anteils an löslicher Guanylatzyklase mit dreiwertigem Hämeisen in einer biologischen Probe verwendet werden. Eine biologische Probe könnte beispielsweise aus Zellen eines Gewebes oder Organs eines Wirbeltiers oder des Menschen bestehen. Diese Zellen könnten aus einem Organismus entnommen oder über Zellkulturtechniken angezüchtet worden sein. Als Spenderorganismen können Individuen mit gesunden oder krankheitsmäßig veränderten Geweben oder Organen ausgewählt werden. Beispielsweise können Organproben, Gewebeproben, Blutproben, Zellen oder Biopsiematerial aus menschlichen oder tierischen Organismen mittels medizinischer Techniken entnommen werden und als biologische Probe dienen. Auch können die biologischen Proben von unterschiedlichen Individuen vergleichend im Hinblick auf deren verschiedene Lebens- und Eßgewohnheiten im Zusammenhang mit dem Oxidationszustand des Hämeisens der löslichen Guanylatzyklase analysiert werden. So ließen sich beispielsweise die Zellen von Rauchern und Nichtrauchern gewinnen und analysieren. Man könnte die lösliche Guanylatzyklasen der Raucher mit derjenigen von Nichtrauchern hinsichtlich von Unterschieden im Verhältnis des zweiwertigen Hämeisens zu dreiwertigem Hämeisen vergleichen.

Die Erfindung offenbart in einer bevorzugten Ausführungsform ein in vitro Verfahren zur Bestimmung der Oxidationsstufe des Hämeisens einer löslichen Guanylatzyklase in einer biologischen Probe, die einem eukaryotischen Organismus entnommen wurde. In der biologischen Probe entnommen aus einem eukaryotischen Organismus können beispielsweise gesunde oder krankheitsmäßig veränderte Zellen aus Zellkulturen, Geweben oder Organen enthalten sein.

In einer weiteren bevorzugten Ausführungsform kann zur Durchführung des in vitro Verfahren ein oben beschriebenes Diagnostikum verwendet werden. Krankheitsmäßig veränderte biologische Zustände können beispielsweise Krebs, Angina pectoris, Diabetes, erektile Dysfunktion, Myokardinfarkt, Herzinsuffizienz, Bluthochdruck, Thrombosen, Anämie, vaskuläre Dysfunktion und andere sein. Es können auch bestimmte Lebens- oder Ernährungsgewohnheiten wie z.B. Rauchen oder Alkoholgenuß auf die Auswirkungen bezüglich des Oxidationszustands des Hämeisens der löslichen Guanylatzyklase untersucht werden.

Bei gesunden biologischen Zuständen fallen die zur Charakterisierung herangezogenen Parameter in den Normalbereich. Entsprechende Angaben zur Festlegung der Normalbereiche finden sich beispielsweise in Lehrbüchern der klinischen Chemie (z.B. Keller, Herbert: Klinisch-chemische Labordiagnostik für die Praxis; Thieme-Verlag, Stuttgart).

Die Erfindung betrifft auch ein Verfahren zum Nachweis einer löslichen Guanylatzyklase, wobei die lösliche Guanylatzyklase ohne Hämgruppe vorliegt. Das Verfahren kann als Verfahrensschritte umfassen a) die Bereitstellung einer löslichen Guanylatzyklase ohne Hämgruppe, b) die Bereitstellung mindestens einer chemischen Verbindung, welche die Aktivität der löslichen Guanylatzyklase stimulieren kann, wenn die lösliche Guanylatzyklase ohne Hämgruppe vorliegt, c) die Inkubation einer löslichen Guanylatzyklase mit einer chemischen Verbindung, welche die Aktivität einer löslichen Guanylatzyklase ohne Hämgruppe stimulieren kann und d) die Bestimmung der Aktivität der löslichen Guanylatzyklase ohne Hämgruppe nach Inkubation mit der chemischen Verbindung gemäß dem luminometrischen Test.

Ein Verfahren zur Bereitstellung einer löslichen Guanylatzyklase ohne Hämgruppe ist beschrieben in Foerster, J. et al., Eur. J. Biochem. 240, 380 - 386 (1996). Als chemische Verbindung zur Stimulierung einer löslichen Guanylatzyklase ohne Hämgruppe eignet sich beispielsweise die chemische Verbindung 2-(4-Chlor-phenylsulfonylamino)-4,5-dimethoxy-N-(4-(thiomorpholin-4-sulfonyl)-phenyl)-benzamid.

Beispiele:

Beispiel 1:

Luminometrisches Nachweisverfahren für die Aktivität der löslichen Guanylatzyklase

Die lösliche Guanylat-Cyclase (sGC) katalysiert die Umwandlung von GTP in cGMP und Pyrophosphat. In Gegenwart von Nicotinamid-adenin-dinucleotid ($NAD^+$) und Nicotinamid-mononucleotid adenylyltransferase (NAT, EC 2.7.7.1) wird Pyrophosphat zu ATP und Nicotinamid-mononucleotid umgesetzt. Das gebildete ATP kann mit Hilfe einer Luciferase luminometrisch in Mikrotiter-Platten quantifiziert werden.

Die zu untersuchende Substanz wird in DMSO gelöst und mit DMSO / Wasser bis zu einer Endkonzentration von 50 μM im Testansatz verdünnt. Die DMSO-Konzentration im Testansatz sollte maximal 5% (v/v) betragen.

100 μl Reaktionsansatz sollten 50 mM TEA-Puffer (pH 7,4), 3mM $MgCl_2$, 3mM GSH, 0,1 mM GTP, 1 mM IBMX (Isobutylmethylxanthin), 0.2 mM $NAD^+$, 0,4 mU NAT, geeignet verdünnte sGC-Enzymlösung (isoliert aus Rinderlunge gemäß Humbert P. et al., Methods of Enzymology 195, 384 -391 (1991)) sowie die Prüfsubstanz oder Lösungsmittel (für die Bestimmung der basalen Enzymaktivität) enthalten. Die Reaktion wird durch Zugabe der sGC gestartet. Der Reaktionsmix wird für 60 min bei Raumtemperatur inkubiert und dann durch Eiskühlung und Zugabe von 50 mM EDTA, pH 8,0 gestoppt. Zur ATP-Bestimmung werden 20 μl 100 mM $MgCl_2$ und 50 μl ATP Test Reagenz (0,035 mM Luciferin und 10000 U/mL Luciferase in 62,5 mM Tris-Acetat pH 7,75, 1,9 mM EDTA, 0,05 mM Dithiothreitol, 0,1% BSA) zugegeben. Die relativen Lumineszenz-Einheiten (RLU) können in einem Mikrotiter-Platten Luminometer gemessen werden.

Die Aktivität der sGC wird nach Abzug des RLU-Leerwertes (Inkubation ohne Enzym) vom RLU-Meßwert erhalten. Die Aktivierung der sGC durch eine Prüfsubstanz wird in Prozent der basalen Enzymaktivität (Lösungsmittel-Kontrolle) angegeben und nach der folgenden Formel berechnet:

$$100 \times (\Delta RLU_{\text{Prüfsubstanz}} / DRLU_{\text{Kontrolle}}) - 100 = \quad \% \text{ Aktivierung}$$

Beispiel 2:

Aktivierung der löslichen Guanylatzyklase nach Oxidation des Hämeisens

Isolierte sGC liegt in Lösung in einer Häm-$Fe^{2+}$-haltigen (reduzierten), NO-stimulierbaren und einer Häm-$Fe^{3+}$-haltigen (oxidierten), nicht NO-stimulierbaren Form vor. Der Anteil der jeweiligen Form kann bisher mit Hilfe von ESR-Messungen bestimmt werden.

Stickstoff-Monoxid (NO) und NO-Donoren aktivieren ausschließlich den reduzierten Zustand der sGC. Sie zeigen bei einer Inkubation in Gegenwart von z. B. 0,01 mM 1H- [1,2,4]oxadiazolo[4,3-a]quinoxalin-1-one (ODQ), das in vitro irreversibel die sGC oxidiert, einen kompletten Verlust ihrer stimulierenden Wirkung. Ein neuer NOunabhängiger Aktivator der sGC, 1-Benzyl-3-(2-hydroxymethyl-fur-5-yl)-indazol (YC-1) aktiviert ebenfalls die reduzierte Form der sGC und ist durch ODQ hemmbar.

Im Gegensatz dazu wird die Stimulierbarkeit durch Substanzen, die ausschließlich oder vorwiegend die oxidierte Form aktivieren, durch ODQ entweder verstärkt oder nicht beeinflußt, je nach dem Anteil der oxidierten Form im Ansatz.

Häufig zeigen diese Substanzen ebenfalls eine Aktivierung der hämfreien Form der sGC. Die Herstellung einer hämfreien sGC wird nach J. Foerster, et al., Eur. J. Biochem. 240, 380-386 (1996) durchgeführt.

Eine lösliche Guanylatzyklase mit einem dreiwertigen Hämeisen läßt sich herstellen, indem frisch präpariertes Enzym für ca. 7 Tage bei 4°C gelagert wird. Inkubiert man auf diese Weise vorbehandelte lösliche Guanylatzyklase mit 2-(4-Chlor-phenylsulfonylamino)-4,5-dimethoxy-N-(4-(thiomorpholin-4-sulfonyl)-phenyl)-benzamid erhält man eine 17,6 fache Stimulierung der Aktivität. Der EC50 Wert beträgt 0,5 μmol/l. Verwendet man eine hämfreie lösliche Guanylatzyklase, erreicht man eine 58,2 fache Stimulierung. Die Affinität liegt in diesem Fall bei 2,4 μmol/l.

Beispiel 3:

Nachweis der Stimulation der löslichen Guanylatzyklase nach Oxidation des Hämeisens durch Bestimmung der Wirkung auf die Aggregation von Thrombozyten

Die physiologische Wirkung von Stickstoff-Monoxid (NO) wie z. B. Hemmung der Thrombozyten-Aggregation und Relaxation der glatten Gefäßmuskulatur ist die Folge einer direkten Aktivierung der löslichen (reduzierten) Guanylatcyclase durch NO unter vermehrter Bildung von cGMP.

ODQ, ein selektiver Inhibitor der sGC, hemmt durch die Oxidation der sGC die cGMP-Erhöhung und die antiaggregatorische Wirkung von NO-Donoren in gewaschenen menschlichen Thrombozyten (M.A. Moro et al., PNAS 93, 1480-1485 (1996)).

Für die Herstellung von gewaschenen humanen Thrombozyten (WP) wird Blut aus der Antecubitalvene entnommen und mit Zitronensäure/Natriumcitrat in der Spritze antikoaguliert. Nach Zentrifugation bei 16 x g für 15 Min. besteht der Überstand aus Plättchen-reichem Plasma (PRP). Das PRP wird angesäuert, die Thrombozyten durch 20 Min. Zentrifugation bei 400 x g sedimentiert und in Tyrode-Lösung aufgenommen. Diese gewaschenen Thrombozyten (WP, $3 \times 10^5/\mu L$) werden in den Versuchen eingesetzt.

Die Hemmung der Aggregation durch sGC Aktivatoren wird in einem Lumiaggregometer bestimmt. Die WPs werden in Gegenwart von 0,5 mM $CaCl_2$ mit der Prüfsubstanz bei 37°C inkubiert und die Reaktion durch Zugabe von z. B. 0,3 µg/mL Collagen gestartet. Die Wirkung der Substanzen auf die Aggregation wird in Abwesenheit und Gegenwart von ODQ untersucht. Aktivatoren der sGC sollten die Collagen-induzierte Thrombozyten-Aggregation Dosis-abhängig hemmen. Aktivatoren, die die oxidierte Form der sGC stimulieren, sollten in Gegenwart von ODQ eine stärkere antiaggregatorische Wirkung zeigen. Der $IC_{50}$ Wert für die Hemmung der Aggregation sollte zu kleineren Konzentrationen verschoben werden.

Zur Bestimmung der intrazellulären cGMP-Konzentration werden WPs 15 Min. in Gegenwart von 0,1 mM Isobutyl-methylxanthin (IBMX) bei 37°C inkubiert. Die Inkubation wird durch Zentrifugation gestoppt und der Niederschlag mit 1 M Perchlorsäure und Ultraschall für 1 Min. behandelt. Nach erneuter Zentrifugation für 15 Min. bei 13000 x g wird der Überstand mit 1 M KOH neutralisiert und die cGMP-Konzentration mit einem käuflichen Enzym-Immuno-Assay (z. B. Amersham) für cGMP bestimmt. Die Proteinkonzentration wird im Niederschlag mit der Bradford-Methode bestimmt.

Chemische Verbindungen, welche die Aktivität der löslichen Guanylatzyklase stimulieren, sollten zu einer Konzentrations-abhängigen Erhöhung der intrazellulären cGMP-Konzentration führen. Wird die Inkubation in Gegenwart von ODQ durchgeführt, sollte mit chemischen Verbindungen, welche die Aktivität der löslichen Guanylatzyklase mit dreiwertigem Hämeisen stimulieren, signifikant höhere cGMP-Spiegel erhalten werden können als ohne ODQ, d. h. ODQ sollte den Effekt der Substanzen verstärken.

Beispiel 4:

Nachweis der Stimulation der löslichen Guanylatzyklase nach Oxidation des Hämeisens durch Bestimmung der Wirkung auf Glattmuskelzellen aus Rattenaorta

Glattmuskelzellen (VSMC) aus Rattenaorta werden nach der Methode von J.H. Chamley et al., Cell Tissue Res. 177, 503-522 (1977) isoliert und kultiviert.

Die Zellen werden in 6-well Platten ausgesät und für 15 Min. in Hepes-Tyrode-Puffer (pH 7,4 mit 200 U SOD, 0,3 mM IBMX) bei 37°C inkubiert. Die Wirkung der Substanzen auf die VSMC wird in Abwesenheit und Gegenwart von ODQ untersucht. Die Inkubation wird durch Absaugen des Überstandes und Zugabe von flüssigem Stickstoff gestoppt und die Zellen werden in den 6-well Platten tiefgefroren. Zur cGMP-Bestimmung werden die Platten aufgetaut, die einzelnen Wells mit Puffer überschichtet und im Überstand die cGMP-Konzentration mit einem käuflichen Enzym-Immuno-Assay (z. B. Amersham) für cGMP bestimmt. Die Proteinkonzentration wird nach Auflösung des Proteins in den Wells mit 0,1 M NaOH mit der Bradford-Methode bestimmt.

Chemische Verbindungen, welche die Aktivität der löslichen Guanylatzyklase stimulieren, sollten zu einer Konzentrations-abhängigen Erhöhung der intrazellulären cGMP-Konzentration führen. Wird die Inkubation in Gegenwart von ODQ durchgeführt, sollten mit chemischen Verbindungen, welche die Aktivität der löslichen Guanylatzyklase mit einem dreiwertigen Hämeisen stimulieren wesentliche höhere cGMP-Spiegel erhalten können. ODQ sollte den Effekt der aktivierenden Verbindungen verstärken.

Beispiel 5:

Herstellung von 2-(4-Chlor-phenylsulfonylamino)-4,5-dimethoxy-N-(4-(thiomorpholin-4-sulfonyl)-phenyl)-benzamid

33.71 g (0.32 mol) Natriumcarbonat wurden in 250 ml Wasser gelöst und auf 60 °C erwärmt. In die Lösung wurden 25.00 g (0.13 mol) 2-Amino-4,5-dimethoxybenzoesäure eingetragen und zu dieser Lösung im Zeitraum von 15 min 29.55 g (0.14 mol) 4-Chlor-benzolsulfonylchlorid portionsweise zugegeben. Nach Abkühlen des Gemisches wurde abgesaugt, der Rückstand in 1 %iger Natriumhydrogencarbonatlösung aufgenommen, filtriert und durch Zugabe von 1 N Salzsäure das Produkt gefällt. Man erhielt 25.90 g (55 %) 2-(4-Chlor-phenylsulfonylamino)-4,5-dimethoxy-benzoesäure vom Schmelzpunkt 212-214 °C. 25.90 g (0.07 mol) 2-(4-Chlor-phenylsulfonylamino)-4,5-dimethoxy-benzoesäure wurden in 75 ml Toluol angeschlämmt, 17.30 g (0.08 mol) Phosphorpentachlorid zugesetzt und das Gemisch 2.5 h bei 40-45 °C gerührt. Anschließend wurde im Vakuum auf das halbe Volumen eingeengt, das ausfallende Produkt abgesaugt und mit wenig Toluol nachgewaschen. Man erhielt 25.30 g (93 %) 2-(4-Chlor-phenylsulfonylamino)-4,5-dimethoxy-benzoesäurechlorid vom Schmelzpunkt 175-177°C.

10.00 g (25.6 mmol) 2-(4-Chlor-phenylsulfonylamino)-4,5-dimethoxybenzoesäurechlorid wurden in 300 ml Toluol angeschlämmt, 4.49 g (25.6 mmol) 4-Aminobenzolsulfonsäurefluorid zugesetzt und das Gemisch 4 h unter Rückfluß erhitzt. Nach Abkühlen wurde der ausgefallene Niederschlag abgesaugt und mit Toluol gewaschen. Man erhielt 11.71 g (87 %) der Titelverbindung vom Schmelzpunkt 216-219 °C.

500 mg (0.95 mmol) 4-((2-(4-Chlor-phenylsulfonylamino)-4,5-dimethoxy-benzoyl)-amino)-benzolsulfonsäurefluorid wurden in 1 ml Thiomorpholin gelöst und 30 min auf 90 °C erhitzt. Zur Aufarbeitung wurde auf 50 ml Eis/1N Salzsäure gegossen, der Niederschlag abgesaugt, im Vakuumtrockenschrank über Phosphorpentoxid getrocknet und aus Hexan/Essigester umkristallisiert. Man erhielt 378 mg (65 %) der Titelverbindung vom Schmelzpunkt 241 °C.

**Patentansprüche**

1. Luminometrischer Test zum Nachweis einer löslichen Guanylatzyklase umfassend die Verfahrensschritte

   a] Bereitstellung einer löslichen Guanylatzyklase und GTP als Substrat und Umsetzung des GTP zu cGMP und Pyrophosphat;

   b] Bereitstellung einer Nicotinamid-Mononukleotid-Adenyltransferase und Nicotinamiddinukleotid (NAD$^+$) und Umsetzung mit dem Pyrophosphat aus a] zu ATP;

   c] Bereitstellung von Luziferase und Luziferin und luminometrische Bestimmung des in b] gebildeten ATP durch Umsetzung mit Luziferin und Luziferase.

2. Luminometrischer Test zum Nachweis einer löslichen Guanylatzyklase gemäß Anspruch 1, wobei GTP, NAD$^+$, Nicotinamid-Mononukleotid-Adenyltransferase, Luziferin und Luziferase in einem Reaktionsgefäß vorgelegt werden, die Reaktion durch Zugabe der löslichen Guanylatzyklase gestartet wird, die Menge an gebildetem ATP gemessen und daraus die Aktivität der löslichen Guanylatzyklase bestimmt wird.

3. Verwendung eines luminometrischen Tests nach Anspruch 1 oder 2 zum Nachweis einer löslichen Guanylatzyklase zur Bestimmung der Aktivität der löslichen Guanylatzyklase.

4. Verwendung eines luminometrischen Tests gemäß Anspruch 1, 2 oder 3 zum Nachweis einer löslichen Guanylatzyklase deren Eisen des Häm in der dreiwertigen Oxidationsstufe vorliegt.

**Claims**

1. A luminometric assay for detecting a soluble guanylate cyclase comprising the method steps of

   a) provision of a soluble guanylate cyclase and GTP as substrate and conversion of the GTP to cGMP and pyrophosphate;

   b) provision of a nicotinamide-mononucleotide adenyl transferase and nicotinamide dinucleotide (NAD$^+$) and reaction with the pyrophosphate from a) to give ATP;

   c) provision of luciferase and luciferin and luminometric determination of the ATP formed in b) by reaction with luciferin and luciferase.

2. The luminometric assay for detecting a soluble guanylate cyclase as claimed in claim 1, where GTP, NAD$^+$, nicotinamide-mononucleotide adenyl transferase, luciferin and luciferase are introduced into a reaction vessel, the reaction is started by adding the soluble guanylate cyclase, the amount of ATP formed is measured, and the activity of the soluble guanylate cyclase is determined therefrom.

3. The use of a luminometric assay as claimed in claim 1 or 2 for detecting a soluble guanylate cyclase for determining the activity of the soluble guanylate cyclase.

4. The use of a luminometric assay as claimed in claim 1, 2 or 3 for detecting a soluble guanylate cyclase whose iron of the hem is in the trivalent oxidation state.

**Revendications**

1. Essai luminométrique pour la détection d'une guanylate cyclase soluble, comprenant les étapes de processus

   a] production d'une guanylate cyclase soluble et de GTP en tant que substrat et conversion du GTP en cGMP et pyrophosphate ;

   b] production d'une nicotinamide-mononucléotide adényltransférase et de nicotinamide-dinucléotide (NAD⁺) et réaction avec le pyrophosphate provenant de a] conduisant à ATP ;

   c] production de luciférase et de luciférine et détermination luminométrique de l'ATP formé en b] par réaction avec la luciférine et la luciférase.

2. Essai luminométrique pour la détection d'une guanylate cyclase soluble selon la revendication 1, dans lequel on dispose au préalable dans un récipient de réaction GTP, NAD⁺, de la nicotinamide-mononucléotide adényl-transférase, de la luciférine et de la luciférase, on déclenche la réaction par addition de la guanylate cyclase soluble, on mesure la quantité d'ATP formé et on détermine à partir de celle-ci l'activité de la guanylate cyclase soluble.

3. Utilisation d'un essai luminométrique selon la revendication 1 ou 2, pour la détection d'une guanylate cyclase soluble, pour la détermination de l'activité de la guanylate cyclase soluble.

4. Utilisation d'un essai luminométrique selon la revendication 1, 2 ou 3, pour la détection d'une guanylate cyclase soluble dont le fer de l'hème se trouve au degré d'oxydation trivalent.